Europäisches Patentamt

European Patent Office

Office européen des brevets

(19)

(11) Veröffentlichungsnummer: **0 077 959**
**B1**

(12) **EUROPÄISCHE PATENTSCHRIFT**

(45) Veröffentlichungstag der Patentschrift:
05.02.86

(21) Anmeldenummer: **82109371.3**

(22) Anmeldetag: **09.10.82**

(51) Int. Cl.⁴: **C 09 C 3/00**

(54) Transparente farbige Pigmente.

(30) Priorität: **26.10.81 JP 170148/81**

(43) Veröffentlichungstag der Anmeldung:
**04.05.83 Patentblatt 83/18**

(45) Bekanntmachung des Hinweises auf die Patenterteilung:
**05.02.86 Patentblatt 86/6**

(84) Benannte Vertragsstaaten:
**BE CH DE FR GB IT LI NL**

(56) Entgegenhaltungen:
**FR - A - 1 326 901**
**FR - A - 2 072 300**
**US - A - 3 647 492**

(73) Patentinhaber: **Merck Patent Gesellschaft mit beschränkter Haftung, Frankfurter Strasse 250, D-6100 Darmstadt (DE)**

(72) Erfinder: **Watanabe, Takaji, 314, Kamikomachi Ohmiya-city, Saitama Pref., 330 (JP)**
Erfinder: **Noguchi, Tamio, 851-10, Tsumada Atsugi-city, Kanagawa Pref., 243 (JP)**

LIBER, STOCKHOLM 1986

## Beschreibung

Die Erfindung betrifft transparente, farbige Pigmente auf der Basis von plättchenförmigen transparenten Substraten wie Glimmer, Talk oder Glas, die mit farbigen Metalloxiden oder Metallhydroxiden überzogen sind.

Es werden bereits viele Arten von farbigen Pigmenten benutzt bei der Herstellung von Lacken, Druckfarben, Kunststoffen, Kosmetika und anderen Erzeugnissen. Fast alle dieser Pigmente sind jedoch undurchsichtig. Es gibt zwar einige organische Pigmente, die relativ transparent sind und auch eine gute Farbkraft besitzen. Diese Pigmente besitzen jedoch Nachteile in bezug auf ihre geringe Hitze- und Wetterbeständigkeit und ihren hohen Preis. Daneben können viele organische Pigmente nicht ohne Bedenken in der Kosmetik oder der Lebensmittelverpackung eingesetzt werden und sind in ihrer Transparenz nicht befriedigend. Organische Pigmente, deren Transparenz durch eine Oberflächenbeschichtung mit Aluminiumoxid oder Bariumsulfat verbessert wurde, sind insbesondere in der Kosmetik nur begrenzt einsatzfähig, da sie eine ungenügende Dispergierbarkeit, schlechten Glanz und ungenügende Füll- und Hafteigenschaften besitzen.

Es bestand deshalb ein Bedürfnis nach transparenten Farbpigmenten mit verbesserter Dispergierbarkeit, besserem Glanz, klarerer Farbe und verbesserter Eitze- und Wetterbeständigkeit. Insbesondere sollten jedoch auch die Füllund Hafteigenschaften für die Anwendung in der Kosmetik verbessert werden.

Es wurde nun gefunden, daß solche Pigmente hergestellt werden können auf der Basis von plättchenförmigen, transparenten Substraten, die mit einer Metalloxid- oder Metallhydroxidschicht überzogen sind, wobei die Verbesserung dadurch bewirkt werden kann, daß der Überzug zusätzlich ein Erdalkalimetall enthält.

In der US-A-3647492 werden Pigmente beschrieben, wobei ein Perlglanzpigment zusätzlich mit einem Farbpigment beschichtet wird und wobei die dabei aufgebrachten Farbpigmentpartikel mit kilfe eines klebenden Bindemittels auf dem Perlglanzpigment fixiert werden. Als klebende Bindemittel sind u. a. auch fettsaure Salze von Magnesium und Calcium genannt. Dieser Publikation konnte jedoch nicht die Anregung entnommen werden, die an sich bekannte Auffällung von Metalloxiden auf plättchenförmige Substrate, die auch ohne klebende Bindemittel zu fest haftenden Überzügen führt, in Gegenwart einer Erdalkaliverbindung durchzuführen, die dabei zumindest teilweise als Oxid bzw. Hydroxid in die Metalloxidschicht eingebaut wird, wobei Pigmente und verbesserte Eigenschaften erhalten werden.

Gegenstand der Erfindung sind daher transparente, farbige Pigmente auf der Basis von plättchenförmigen, transparenten Substraten wie Glimmer, Talk oder Glas, die mit farbigen Metalloxiden oder Metallhydroxiden überzogen sind, die dadurch gekennzeichnet sind, daß die Metalloxid- bzw. -hydroxidschicht 0,1 - 5 Gew.% eines Erdalkalimetalloxids bzw. -hydroxids enthält.

Gegenstand der Erfindung ist auch ein Verfahren Zur Herstellung von transparenten, farbigen Pigmenten, wobei ein plättchenförmiges, transparentes Substrat in wäßriger Suspension in Gegenwart einer Base mit einem farbigen Metalloxid bzw. -hydroxid beschichtet wird und danach abgetrennt, gegebenenfalls gewaschen, getrocknet und gegebenenfalls kalziniert wird, das dadurch gekennzeichnet ist, daß die Metalloxid- bzw. -hydroxidfällung in Gegenwart einer solchen Menge einer Erdalkalimetallverbindung durchgeführt wird, daß sich in der Metalloxid- bzw. -hydroxidschicht ein Gehalt von 0,1 - 5 Gew.% eines Erdalkalimetalloxids bzw. -hydroxids ergibt.

Als plättchenförmige, transparente Substrate sind geeignet Glimmer, wie z. B. Muscovit, Sericit oder Phlogopit, Talk- und Glasplättchen. Diese Substrate werden in der Regel in Teilchengrößen von etwa 1 - 100 μm eingesetzt, vorzugsweise in einer Größe uon etwa 5 - 50 μm.

Als farbige Metalloxide bzw. -hydroxide geeignet sind z. B. $FeO(OH)$, $Fe(OH)_2$, $Fe(OH)_3$, $Co(OH)_2$, $Cr(OH)_3$, $Fe_2O_3$, $Fe_3O_4$, $CoO$ oder $Cr_2O_3$. Gegebenenfalls können auch farblose Metalloxide, wie z. B. $Al_2O_3$, $ZnO$ oder $TiO_2$, mit aufgefällt werden. Als Erdalkalimetallverbindungen werden vorzugsweise Calciumoxid und -hydroxid und/oder Magnesiumoxid und -hydroxid eingesetzt.

Alle genannten Metalloxide bZw. -hydroxide können sowohl allein als auch gemeinsam in der Schicht vorliegen, wobei sich auch Mischoxide dieser Metalle bilden können. Entscheidend ist lediglich, daß etwa 0,1 - 5 Gew.% der Erdalkaliverbindungen enthalten sind. Unterhalb 0,1 Gew.% werden keine guten Ergebnisse erzielt. Durch einen Gehalt von mehr als 5 Gew.% können die Eigenschaften nicht mehr weiter verbessert werden.

Zur Herstellung der erfindungsgemäßen Pigmente wird das plättchenförmige, transparente Substrat in einer wäßrigen Lösung suspendiert, die sowohl zumindest ein Metallsalz als auch ein Erdalkalimetallsalz enthält. Als geeignete Metallsalze zur Bildung farbiger Oxide bzw. Hydroxide sind z. B. geeignet $FeSO_4$, $FeCl_2$, $F(NH_4)_2(SO_4)_2$, $Fe(NO_3)_2$, $Fe_2(SO_4)_3$, $FeCl_3$, $FeNE_4(SO_4)_2$, $Fe(NO_3)_3$, $CoCl_2$, $CoSO_4$, $Co(NO_3)_2$, $CrCl_3$, $CrNE_4(SO_4)_2$. Als Metallsalze, die zur gegebenenfalls zusätzlich erfolgenden Abscheidung von farblosen Metalloxiden geeignet sind, wären beispielhaft Zu nennen: $Al_2(SO_4)_3$, $AlNH_4(SO_4)_2$, $AlNa(SO_4)_2$, $AlK(SO_4)_2$, $EnCl_2$, $EnSO_4$, $TiOSO_4$, $TiCl_4$. Als Erdalkalimetallsalze sind beispielsweise geeignet: $MgSO_4$, $Mg(NO_3)_2$, $MgCl_2$, $MgBr_2$, $MgJ_2$, $GaCl_2$, $Caßr_2$ und $CaJ_2$. Besonders bevorzugt werden $MgSO_4$, $MgCl_2$ und $CaCl_2$ verwendet.

Alle diese Metall- und Erdalkalimetallsalze können in Kombination miteinander verwendet werden, wobei insbesondere bei der Beschichtung mit den farbigen Oxiden bzw. Hydroxiden die Mengenverhältnisse unterschiedlicher Metallsalze und das Gewichtsverhältnis Beschichtung zu Substrat entsprechend der gewünschten Farbe frei gewählt werden kann.

In der Regel wird eine etwa 5 - 25 Gew.%ige Suspension des plättchenförmigen Substrats in der Lösung der Metallsalze, die etwa $9 \times 10^{-7}$ bis $19 \times 10^{-16}$ mol Metallsalze pro qm zu beschichtender Substratoberfläche enthält, hergestellt. Durch Zugabe einer Base werden die Salze dann hydrolysiert und die Metalle einschließlich der Erdalkalimetalle in Form der Hydroxide bzw. Oxidhydrate aus dem Substrat abgeschieden. Als Base sind geeignet z. B. wäßriger Ammoniak, Ammoniumhydrogencarbonat, Natriumhydrogencarbonat, Kaliumhydrogencarbonat, Natronlauge oder Kalilauge. Daneben können aber auch Stoffe, wie Harnstoff, Acetamid oder Formamid als Basen verwendet werden, die durch Hydrolyse in der wäßrigen Suspension Ammoniak erzeugen. Harnstoff ist besonders bevorzugt, da hierbei eine besonders feine Teilchengröße der auf das Substrat ausgefällten Metalloxidhydrate erzielt werden kann und dadurch eine besonders gute Transparenz der Produkte resultiert. Daneben wird durch Harnstoff eine besonders homogene Abscheidung der Metalloxidhydrate auf dem Substrat erreicht.

Die Base wird als etwa 2 - 10 Gew.%ige Lösung innerhalb etwa 0,5 - 2 Stunden zugegeben, bis die Suspension neutral ist. Danach sollte noch für etwa 30 Minuten nachgerührt werden. Falls Harnstoff als Base verwendet wird, werden etwa 3 - 30 Äquivalente Harnstoff, bezogen auf die Metalloxide, zugesetzt, und die Suspension wird für etwa 0,5 - 5 Stunden gekocht.

Auf diese Weise werden z. B. Magnesiumhydroxid, Calciumhydroxid, Eisen(II)hydroxid, Eisen(III)hydroxid, Kobalthydroxid, Chromhydroxid, Aluminiumhydroxid, Zinkhydroxid, Titanhydroxid Titanoxidhydrat oder Eisenoxidhydrat abgeschieden Nach Beendigung der Abscheidung werden die Produkte abgetrennt, in der Regel gewaschen und bei etwa 100 - 120 °C getrocknet. Gegebenenfalls kann auch eine Kalzinierung erfolgen, die z. B. bei etwa 400 - 900 °C für etwa 0,5 - 5 Stunden durchgeführt wird und bei der die Hydroxide bzw. Oxidhydrate in die Oxide überführt werden.

Da mit den genannten Metalloxiden mit Ausnahme von Aluminium, Zink und Titan eine Reihe verschieden gefärbter Metalloxide zur Verfügung stehen, können durch die Auswahl der Art der Metalloxide bzw. durch Auswahl des Mischungsverhältnisses verschiedener Metalloxide sowohl viele verschiedene Farben, wie z. B. Rot, Gelb, Blau, Grün und Schwarz, sowie neutrale Färbungen dazwischen, als auch sehr viele Farbtönungen hergestellt werden.

Zur Herstellung eines gelben, transparenten Pigmentes wird eine etwa 5 - 25 Gew.%ige wäßrige Suspension des plättchenförmigen Substrates bereitet, die etwa 10 - 150, vorzugsweise etwa 30 - 90 Teile eines Eisensalzes, 150, vorzugsweise 2 - 30 Teile eines Erdalkalimetallsalzes und 10 - 400, vorzugsweise 30 - 270 Teile Harnstoff, jeweils bezogen auf 100 Teile des Substrats, gelöst enthält. Diese Suspension wird für etwa 0,5 - 5 Stunden aufgekocht, wonach ein gelbes Pigment durch Abtrennen, Auswaschen und Trocknen bei etwa 105 - 110 °C gewonnen werden kann.

Durch Erhitzen auf 300 - 1200 °C, vorzugsweise 400 bis 900 °C, kann daraus ein rotes, transparentes Pigment hergestellt werden.

Zur Herstellung eines schwarzen, transparenten Pigments wird eine etwa 5 - 25 Gew.%ige wäßrige Suspension des plättchenförmigen Substrates bereitet, die etwa 50 - 300, vorzugsweise 150 - 250 Teile eines Eisensalzes, 3 - 90, vorzugsweise 15 - 60 Teile eines Erdalkalimetallsalzes, 25 - 1500, vorzugsweise 75 - 1000 Teile Harnstoff und 2,5 - 90, vorzugsweise 8 - 75 Teile Kaliumnitrat, jeweils bezogen auf 100 Teile des Substrats, gelöst enthält. Nach Aufkochen für 0,5 - 5 Stunden, Abtrennen, Waschen und Trocknen bei 100 - 110 °C erhält man ein schwarzes, transparentes Pigment.

Zur Herstellung eines grünen, transparenten Pigments wird eine etwa 5 - 25 Gew.%ige wäßrige Suspension des plättchenförmigen Substrats bereitet, die 20 - 200, vorzugsweise 40 - 150 Teile eines Kobaltsalzes, 20 - 200, vorzugsweise 40 - 150 Teiles eines Aluminiumsalzes, 760, vorzugsweise 10 - 30 Teile eines Erdalkimetallsalzes und 100 - 800, vorzugsweise 200 - 450 Teile Harnstoff, jeweils bezogen auf 100 Teile des Substrats, gelöst enthält. Nach Aufkochen für 0,5 - 5 Stunden, Abtrennen, Waschen und Trocknen bei etwa 120 °C und Kalzinieren bei etwa 500 - 700 °C erhält man ein grünes, transparentes Pigment.

Durch Kalzinieren bei 800 - 1200 °C erhält man daraus ein blaues, transparentes Pigment.

Die so erhaltenen Pigmente sind ausgezeichnet in bezug auf Dispergierbarkeit, Glanz, Farbklarheit und Hitzebeständigkeit und können für zahlreiche Anwendungen, insbesondere in der Kosmetik, verwendet werden.

**Beispiel 1**

Eine Lösung von 55 g Eisen(III)ammoniumsulfat, 10 g Magnesiumsulfat und 80 g Harnstoff in 900 ml Wasser, in der 90 g Glimmer einer Teilchengröße von 1 - 10 μm suspendiert sind, wird für eine Stunde auf 95 - 98 °C erhitzt. Danach wird das Produkt abgetrennt, mit Wasser gewaschen und bei 105 - 110 °C getrocknet. Man erhält ein Pigment mit der gelben Farbe des Eisenoxidhydroxids, das gute Transparenz und Dispergierbarkeit besitzt.

**Beispiel 2**

Eine Lösung von 7,5 g Eisen(III)ammoniumsulfat, 1,5 g Magnesiumsulfat und 20 g Harnstoff in 100 ml Wasser, in der 10 g Talk suspendiert sind, wird für 1 Stunde auf 9598 °C erhitzt. Das gebildete Produkt wird abgetrennt, mit Wasser gewaschen und bei 105 - 110 °C getrocknet. Das gebildete Pigment, ein auf Talk abgeschiedenes Eisenoxidhydroxid zeigt eine gelbe Farbe und besitzt eine gute Dispergierbarkeit.

**Beispiel 3**

Eine Lösung von 80 g Eisen(III)ammoniumsulfat, 18 g Magnesiumsulfat und 162 g Harnstoff in 900 ml Wasser, die 90 g Glimmer einer Teilchengröße von 1 - 10 μm suspendiert enthält, wird für eine Stunde auf 95 - 98 °C erhitzt. Das Produkt wird abgetrennt, mit Wasser gewaschen und bei 105 - 110 °C getrocknet. Das gebildete Pigment ist gelbocker und besitzt gute Transparenz und Dispergierbarkeit.

**Beispiel 4**

Eine Lösung von 64 g Eisen(III)chlorid, 13 g Magnesiumsulfat und 128 g Harnstoff in 900 ml Wasser, die 90 g Glimmer einer Teilchengröße von 1 - 10 μm dispergiert enthält, wird für 2 Stunden auf 95 - 98 °C erhitzt. Danach wird abfiltriert, gewaschen und für 8 Stunden bei 105110 °C getrocknet. Man erhält ein gelb-ockerfarbiges Pigment mit guter Transparenz und Dispergierbarkeit.

**Beispiel 5**

Das nach Beispiel 4 hergestellte Pigment wird 1 Stunde bei 700 °C kalziniert. Man erhält ein rotes, transparentes Pigment.

**Beispiel 6**

Eine Lösung von 128 g Eisen(III)chlorid, 26 g Magnesiumsulfat und 256 g Harnstoff in 900 ml Wasser, die 90 g Glimmer einer Teilchengröße von 1 - 10 μm dispergiert enthält, wird für 2 Stunden auf 95 - 98 °C erhitzt. Nach Filtrieren, Waschen mit Wasser und Trocknen für 8 Stunden bei 105 - 110 °C erhält man ein gelb-ockerfarbiges Pigment mit guter Transparenz und Dispergierbarkeit.

**Beispiel 7**

Das nach Beispiel 6 hergestellte Pigment wird für eine Stunde bei 700 °C kalziniert. Man erhält ein violettrotes, transparentes Pigment.

**Beispiel 8**

Eine Lösung von 178 g Eisen(II)sulfat, 40 g Magnesiumsulfat, 32 g Kaliumnitrat und 300 g Harnstoff in 900 ml Wasser, die 80 g Glimmer einer Teilchengröße von 1 bis 10 μm dispergiert enthält, wird für 4 Stunden auf 9598 °C erhitzt. Nach Filtrieren, Waschen mit Wasser und Trocknen für 10 Stunden bei 105 - 110 °C erhält man ein schwarzes Pigment mit guter Transparenz und Dispergierbarkeit.

**Beispiel 9**

Eine Lösung von 3,0 g Aluminiumchlorid, 3,0 g Kobaltchlorid, 1,0 g Magnesiumsulfat und 20 g Harnstoff in 60 ml Wasser, die 5,0 g Glimmer einer Teilchengröße von 1 - 10 μm dispergiert enthält, wird für 2 Stunden auf 95 - 98 °C erhitzt. Das Produkt wird abfiltriert, mit Wasser gewaschen und für 2 Stunden bei 105 - 110.°C getrocknet. Nach Kalzinieren des gebildeten weißlichen Produktes für eine Stunde bei 600 °C erhält man ein transparentes Pigment.

**Beispiel 10**

Das nach Beispiel 9 hergestellte weißliche Pigment wird für eine Stunde bei 800 °C kalziniert. Man erhält ein blaues, transparentes Pigment.

**Beispiel 11**

Eine Lösung von 20 g Eisen(III)chlorid in 300 ml Wasser, die 30 g Glimmer einer Teilchengröße von 1 - 10 μm dispergiert enthält, wird eine Stunde auf 78 - 82 °C erhitzt und danach innerhalb von 0,5 Stunden mit 85 ml einer 10%igen Natronlauge versetzt. Zu der Lösung, die einen pHWert von 2,3 aufweist, wird innerhalb von 5 Minuten eine 6%ige Calciumchloridlösung zugegeben und danach so viel 10%ige Natronlauge, daß ein neutraler pH-Wert erreicht wird. Das Produkt wird abgetrennt, mit Wasser gewaschen und 2 Stunden bei 105 - 110 °C getrocknet. Nach Kalzinieren bei 700 °C für eine Stunde erhält man ein rotes, transparentes Pigment.

## Beispiel 12

Eine Lösung von 3,0 g Chrom(III)sulfat, 1,0 g Magnesiumsulfat und 10,0 g Harnstoff in 150 ml Wasser, die 10 g Glimmer einer Teilchengröße von 1 -10 µm dispergiert enthält, wird für 0,5 Stunden auf 95 - 98 °C erhitzt. Das Produkt wird abfiltriert, mit Wasser gewaschen und 5 Stunden bei 98 - 105 °C getrocknet. Nach Kalzinieren des hellgrünen Produktes bei 800 °C für eine Stunde erhält man ein blau-grünes, transparentes Pigment.

## Beispiel 13

Eine Lösung von 2,0 g Zinkchlorid, 2,0 g Eisen(III)chlorid, 1,0 g Magnesiumsulfat und 20 g Harnstoff in 100 ml Wasser, die 5,0 g Glimmer einer Teilchengröße von 1 bis 10 µm dispergiert enthält, wird für eine Stunde auf 95 bis 98 °C erhitzt. Das Produkt wird abfiltriert, mit Wasser gewaschen und für 10 Stunden bei 98 - 105 °C getrocknet. Nach Kalzinieren des Produktes bei 800 °C für eine Stunde erhält män ein gelb-ockerfarbiges, transparentes Pigment.

## Beispiel 14

Eine Suspension von 5 g Glimmer einer Teilchengröße von 1 - 10 µm in 50 ml Wasser wird für 0,5 Stunden auf 9598 °C erhitzt und danach innerhalb von 80 Minuten mit einer Lösung von 20 g Titansulfat, 4 g Magnesiumsulfat und 40 g Harnstoff in 100 ml Wasser versetzt und noch für eine Stunde gerührt. Nach Kalzinieren des dabei gewonnenen weißlichen Produktes bei 800 °C für 1 Stunde erhält man ein transparentes Pigment mit blau-grüner Interferenzfarbe.

## Vergleichsbeispiel 1

Eine Lösung von 128 g Eisen(III)chlorid und 256 g Harnstoff in 900 ml Wasser, die 90 g Glimmer einer Teilchengröße von 1 - 10 µm dispergiert enthält, wird für 2 Stunden auf 95 - 98 °C erhitzt. Nach Abfiltrieren, Waschen mit Wasser, Trocknen bei 105 - 110 °C für 8 Stunden und Kalzinieren bei 700 °C für eine Stunde erhält man ein violett-rotes Pigment. Die Dispergierbarkeit dieses Produktes wird mit der des Produktes nach Beispiel 7 nach folgender Methode verglichen: In ein 300 ml-Becherglas werden 45 g einer Standard-Lackgrundlage (VS medium ink) gegeben und 5,0 g des jeweiligen Pigmentes bei einer Drehzahl von 500 U/min eingerührt. Nach jeweils 5, 10, 30 und 60 Minuten werden Proben entnommen und auf Beschichtungspapier, das einen weißen und einen schwarzen Grund besitzt, ausgestrichen. Anhand der so gewonnenen Proben kann die Dispergierbarkeit des jeweiligen Pigments beurteilt werden. Während das Pigment nach Beispiel 7 bereits nach 5 Minuten sehr gut dispergiert ist, ist das Vergleichspigment sogar nach 60 Minuten noch nicht ausreichend dispergiert.

## Vergleichsbeispiel 2

Eine Lösung von 178 g Eisen(II)sulfat, 32 g Kaliumnitrat und 300 g Harnstoff in 900 ml Wasser, die 80 g Glimmer einer Teilchengröße von 1 - 10 µm dispergiert enthält, wird für 4 Stunden auf 95 - 98 °C erhitzt, danach das Produkt abfiltriert, mit Wasser gewaschen und 10 Stunden bei 98 - 105 °C getrocknet. Die Hitzebeständigkeit und die Stabilität gegen Oxidation dieser Probe wird bestimmt durch Messung der TGA- und DTA-Werte. Das Ergebnis zeigt, daß sich die TGA- und DTA-Kurven bei 155 °C verändern, wobei der Farbton des Pigmentes sich von Schwarz nach Rotbraun ändert. Das nach Beispiel 8 hergestellte Pigment dagegen, das Magnesium enthält, zeigte keine Änderung bis zu einer Temperatur von 260 °C.

## Anwendungsbeispiel

Kompaktpuder zur Benutzung in Make-up
Pigment des Beispiels 5: 30 Gew.%
Talk: 52,5 Gew.%
Kaolin: 3 Gew.%
Calciumstearat: 5 Gew.%
Kornstärke: 3 Gew.%
Isopropyllaurat: 5 Gew.%
Isopropylmyristat: 1 Gew.%
Parfüm: 0,5 Gew.%
In dem so hergestellten Kosmetikum zeigt das erfindungsgemäße Pigment eine bessere Transparenz und bessere Füll- und Hafteigenschaften als bisher benutzte Eisenoxidpigmente.

## Patentansprüche

1. Transparente, farbige Pigmente auf der Basis von plättchenförmigen, transparenten Substraten wie Glimmer, Talk oder Glas, die mit farbigen Metalloxiden oder Metallhydroxiden überzogen sind, dadurch gekennzeichnet, daß die Metalloxid- bzw. -hydroxidschicht 0,1 - 5 Gew.% eines Erdalkalimetalloxids bzw. -hydroxids enthält.

2. Verfahren zur Herstellung von transparenten, farbigen Pigmenten, wobei ein plättchenförmiges, transparentes Substrat in wäßriger Suspension in Gegenwart einer Base mit einem farbigen Metalloxid bzw. -hydroxid beschichtet wird und danach abgetrennt, gegebenenfalls gewaschen, getrocknet und gegebenenfalls kalziniert wird, dadurch gekennzeichnet, daß die Metalloxid-
bzw. -hydroxidfällung in Gegenwart einer solchen
Menge einer Erdalkalimetallverbindung
durchgeführt wird, daß sich in der Metalloxid-
bzw. -hydroxidschicht ein Gehalt von 0,1 - 5
Gew.% eines Erdalkalimetalloxids bzw. -
hydroxids ergibt.

3. Verfahren nach Anspruch 2, dadurch
gekennzeichnet, daß die Fällung der Metalloxide
bzw. -hydroxide durch die Gegenwart von
Harnstoff bewirkt wird.

**Revendications**

1. Pigments colorés transparents à base da
substrats transparants en forme de plaquettes
comme la mica, le talc ou le verre, qui sont
recouverts d'oxydes métalliques ou hydroxydes
métalliques colorés, caractérisés en ce que la
couche d'oxyde ou d'hydroxyde métallique
contient de 0,1 à 5% en poids d'un oxyde ou
hydroxyde de métal alcalino-terreux.

2. Procédé de préparation de pigments colorès
transparents, où l'on recouvre un substrat
transparent en forme de plaquettes en
suspension aqueuse an présence d'une base avec
un oxyde ou hydroxyde métallique coloré, puis en
ce qu'on le sépare, éventuellement en ce qu'on le
lave, en ce qu'on le sèche et éventuellement en
ce qu'on le calcine, caractérisé en ce qu'on
conduit la précipitation de l'oxyde ou hydroxyde
métallique en présence d'une quantité de
composé da métal alcalino-terreux telle qu'on ait
dans la couche d'oxyde ou hydroxyde métallique
une teneur de 0,1 - 5% en poids d'un oxyde ou
hydroxyde de métal alcalino-terreux.

3. Procédé selon la revendication 2, caractérisé
en ce que la précipitation des oxydes ou
hydroxydes métalliques est provoquée par la
présence d'urée.

**Claims**

1. Transparent, coloured pigments based on
plateletshaped, transparent substrates, such as
mica, talc or glass, which are coated with
coloured metal oxides or metal hydroxides,
characterised in that the metal oxide or hydroxide
layer contains 0.1 - 5% by weight of an alkaline
earth metal oxide or hydroxide.

2. Process for the preparation of transparent,
coloured pigments in which a platelet-shaped,
transparent substrate is coated, in aqueous
suspension in the presence of a base, with a
coloured metal oxide or hydroxide and is then
separated off, washed if necessary, dried and, if
necessary, calcined, characterised in that the
metal oxide or hydroxide precipitation is carried
out in the presence of such an ammount of an
alkaline earth metal compound that a content of
0.1 - 5% by weight of the alkaline earth metal oxide or hydroxide results in the metal oxide or
hydroxide layer.

3. Process according to Claim 2 characterised
in that the precipitation of the metal oxides or
hydroxides is effected by the presence of urea.